(19) Europäisches Patentamt
European Patent Office
Office européen des brevets

(11) EP 3 034 067 A1

(12) # EUROPEAN PATENT APPLICATION

(43) Date of publication:
**22.06.2016 Bulletin 2016/25**

(51) Int Cl.:
***A61K 8/35*** (2006.01) ***A61K 8/41*** (2006.01)
***A61Q 5/06*** (2006.01)

(21) Application number: **14199499.6**

(22) Date of filing: **19.12.2014**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Unilever PLC**
**London, Greater London EC4Y 0DY (GB)**

(72) Inventors:
- **BATCHELOR, Stephen Norman**
**Merseyside, CH63 3JW (GB)**
- **LIMER, Adam John**
**Merseyside, CH63 3JW (GB)**
- **YANG, Xiaoxia**
**Shanghai, 200335 (CN)**

(74) Representative: **James, Helen Sarah**
**Unilever PLC**
**Unilever Patent Group**
**Colworth House, Sharnbrook**
**Bedford**
**Bedfordshire MK44 1LQ (GB)**

(54) **Hair colouring compositions**

(57) The invention provides a method for the non-oxidative (direct) coloration of hair, the method comprising the step of topically applying a direct dye to the hair in the presence of dihydroxyacetone; the direct dye having a nitrobenzene chromophore and at least one primary amine ($-NH_2$) group.

The presence of dihydroxyacetone increases the direct dye substantivity and resistance to wash off.

EP 3 034 067 A1

## Description

Field of the Invention

This invention relates to non-oxidative (direct) hair colouring compositions comprising at least one nitrobenzene direct dye.

Background of the Invention and Prior Art

Non-oxidative hair dyeing processes are often referred -to as "direct" dyeing because the dyes are applied directly and the molecules remain chemically unchanged within the hair after application.

In contrast to permanent oxidative dyeing, which involves *in situ* chemical reactions, the direct dyeing process does not require the simultaneous or subsequent application of an oxidising agent, such as hydrogen peroxide, to develop the colour.

Nitrobenzene direct dyes provide colours ranging through yellow, orange and red-brown to violet. They have gained wide acceptance as direct hair dyes because of their tinctorial effectiveness and ease of application.

For example, US 3 930 792 describes semi-permanent hair dye compositions incorporating a nitrobenzene direct dye. The compositions incorporate a hair substantive quaternary amine compound and an N-oxyalkylated long chain fatty acid amide. This combination is said to prevent the hair from snarling when wet and to improve combability, manageability, sheen and texture.

One drawback which may be associated with nitrobenzene direct dyes is their insufficient resistance to removal. Repeated washing is the most significant factor in dye removal, and this progressive "washing-out" causes both fading and change of tone of dyed hair. Other sensory characteristics such as shine, body, lustrous feel and manageability are also linked to colour retention.

The present invention provides a solution to this problem.

Summary of the Invention

The present invention provides a method for the non-oxidative (direct) coloration of hair, the method comprising the step of topically applying a direct dye to the hair in the presence of dihydroxyacetone; the direct dye having a nitrobenzene chromophore and at least one primary amine ($-NH_2$) group.

Detailed Description of the Invention

The term "direct dye" in the context of this invention means a coloured molecule which is able to impart colour to the hair by diffusion into the hair fibre and/or adsorption at the hair surface.

The term "chromophore" in the context of this invention means the molecular substructure that is responsible for the spectral selective absorption of electromagnetic radiation.

In dyes with a nitrobenzene chromophore (hereinafter termed "nitrobenzene direct dyes"), substituent groups on the benzene ring serve as electron donor groups, and the $NO_2$ group serves as the electron acceptor group. This system of donor and acceptor groups linked together by the unsaturated bridge that is provided by the benzene ring may serve to move the principal absorption band of the chromophore into the visible region (400 to 700 nm).

Suitable nitrobenzene direct dyes for use in the invention will characteristically have a maximum absorption wavelength in the region from 400 nm to 700 nm, with a molar extinction coefficient at the maximum absorption wavelength of at least 5000 L/mol/cm, preferably at least 10000 L/mol/cm. The molar extinction coefficient may suitably be calculated from the equation a/bc wherein a is the maximum absorbance, b is the path length (cm) and c is the concentration in moles/litre of the dye in a suitable solvent (e.g. ethanol).

Suitable nitrobenzene direct dyes for use in the invention will characteristically have a molecular weight of less than about 1000, more preferably less than about 500 and most preferably from about 100 to about 250.

Suitable nitrobenzene direct dyes for use in the invention may carry a primary amine group directly bonded to the benzene nucleus, or may carry an extranuclear primary amine group, or both.

Preferred nitrobenzene direct dyes for use in the invention carry at least one primary amine group directly bonded to the benzene nucleus.

Typical nitrobenzene direct dyes for use in the invention may be characterised by the following general formula (I):

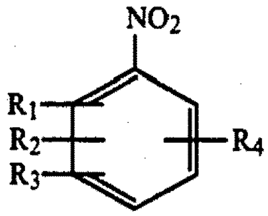

(I)

in which $R_1$ is $-NH_2$ or $-NHCH_2CH_2NH_2$ ;
in which $R_2$,$R_3$ and $R_4$ are each independently selected from hydrogen, halogen, -OH, - $NH_2$, -($C_{1-4}$ alkyl), $-OR_5$, $-NHR_5$ and $-N(R_5)_2$;

in which each $R_5$ is independently selected from $C_{1-4}$ alkyl, monohydroxy ($C_{1-4}$ alkyl), polyhydroxy ($C_{1-4}$ alkyl) and amino ($C_{1-4}$ alkyl) group
and subject to the proviso that at least one of the substituents $R_2$,$R_3$ and $R_4$ is other than hydrogen.

The term "$C_{1-4}$ alkyl" in formula (I) generally denotes a saturated, linear or branched hydrocarbon chain having 1 to 4 carbon atoms. Preferred alkyl groups in this context have 1 or 2 carbon atoms.

$R_1$ in formula (I) is preferably $-NH_2$.

$R_2$,$R_3$ and $R_4$ in formula (I) are preferably each independently selected from hydrogen, Cl, -OH, $-NH_2$, $-CH_3$, $-OCH_3$, $-OCH_2CH_2OH$, $-NHCH_2CH_2OH$, $-OCH_2CH_2NH_2$, $-NHCH_2CH_2NH_2$, $-OCH_2CH(OH)CH_2OH$, $-NHCH_2CH(OH)CH_2OH$, $-NHC(CH_2OH)_3$ and $-N(CH_2CH_2OH)_2$.

More preferably, in formula (I) $R_1$ is $-NH_2$, and $R_2$ and $R_3$ are hydrogen, and $R_4$ is -OH or-$NH_2$ respectively.

Preferred examples of nitrobenzene direct dyes for use in the invention include:

1,4-diamino-2-nitrobenzene
1,2-diamino-4-nitrobenzene
4-amino-3-nitrophenol
2-amino-3-nitrophenol
2-amino-6-chloro-4-nitrophenol
1-amino-3-methyl-4-[(2-hydroxyethyl)amino]-6-nitrobenzene (HC Violet No. 1)
1-amino-4-[(2-hydroxyethyl)amino]-2-nitrobenzene (HC Red No. 7)
1-amino-4-[di-(2-hydroxyethyl)amino]-2-nitrobenzene hydrochloride (HC Red No. 13)
1-amino-5-chloro-4-[(2-hydroxyethyl)amino]-2-nitrobenzene
4-amino-1-[(2-hydroxyethyl)amino]-2-nitrobenzene (HC Red No. 3)
1-[(2-aminoethyl)amino]-4-(2-hydroxyethoxy)-2-nitrobenzene (HC Orange No. 2)
1-amino-5-chloro-4-[(2,3-dihydroxypropyl)amino]-2-nitrobenzene (HC Red No. 10)
1-amino-2-[(2-hydroxyethyl)amino]-5-nitrobenzene (HC Yellow No. 5)
1-amino-2-methyl-6-nitrobenzene
3-[(2-aminoethyl)amino]-1-methoxy-4-nitrobenzene hydrochloride (HC Yellow No. 9)

Mixtures of any of the above described materials may also be used.

The nitrobenzene direct dye for use in the method of the invention will generally be formulated into a dye composition. The dye composition may take various forms, such as a solution, emulsion, cream, gel, paste, mousse, aerosol, or any other form suitable for topical application to the hair.

The dye composition will typically incorporate further ingredients (in addition to the dye) to enhance performance and/or consumer acceptability.

The dye composition will ordinarily be an aqueous composition. The amount of water in such an aqueous dye composition will typically range from about 60 to about 95 wt% and preferably ranges from about 70 to about 90 wt% (by weight based on the total weight of the dye composition).

Other organic solvents may also be present, such as lower alkyl alcohols (e.g. ethanol and isopropanol) and polyhydric alcohols (e.g. propylene glycol, hexylene glycol, glycerin, and propanediol). Mixtures of any of the above described organic solvents may also be used.

In a preferred embodiment, the dye composition may also include one or more hair conditioning ingredients. Exemplary hair conditioning ingredients include cationic surfactants, fatty materials, silicones and mixtures thereof.

Examples of suitable cationic surfactants include quaternary ammonium cationic surfactants corresponding to the following general formula (II):

$$[N(R^1)(R^2)(R^3)(R^4)]^+ (X)^- \qquad (II)$$

in which $R^1$ is a $C_{16}$ to $C_{22}$ saturated or unsaturated, preferably saturated, alkyl chain and $R^2$, $R^3$ and $R^4$ are each independently selected from $CH_3$ and $CH_2CH_2OH$, preferably $CH_3$.

Alternatively, primary, secondary or tertiary fatty amines may be used in combination with an acid to provide a cationic surfactant suitable for use in the invention. The acid protonates the amine and forms an amine salt *in situ.* The amine is therefore effectively a non-permanent quaternary ammonium or pseudo-quaternary ammonium cationic surfactant.

Suitable fatty amines of this type include amidoamines of the following general formula (III):

$$R^1\text{-}C(O)\text{-}N(H)\text{-}R^2\text{-}N(R^3)(R^4) \qquad (III$$

in which $R^1$ is a fatty acid chain containing from 12 to 22 carbon atoms, $R^2$ is an alkylene group containing from one to four carbon atoms, and $R^3$ and $R^4$ are each independently, an alkyl group having from one to four carbon atoms.

Particularly preferred is stearamidopropyldimethylamine.

The acid used may be any organic or mineral acid which is capable of protonating the amine. Preferably, the acid is selected from the group consisting of acetic acid, tartaric acid, hydrochloric acid, fumaric acid, lactic acid and mixtures thereof.

Mixtures of any of the above-described cationic surfactants may also be suitable.

The level of cationic surfactant suitably ranges from about 0.1 to about 10 wt%, preferably from about 0.2 to about 5 wt% and more preferably from about 0.25 to about 4 wt% (by total weight of cationic surfactant based on the total weight of the dye composition).

By "fatty material" is generally meant a compound having the general formula R-X, wherein R is an aliphatic carbon chain and X is a functional group (e.g. alcohol or carboxylic acid or a derivative thereof such as ester or amide).

Preferred fatty materials include fatty alcohols of general formula $CH_3(CH2)n$ OH, where n is an integer from 7 to 29, preferably from 15 to 21. Specific examples of suitable fatty alcohols are cetyl alcohol, stearyl alcohol, behenyl alcohol, and mixtures thereof.

The level of fatty material suitably ranges from about 0.01 to about 10 wt%, preferably from about 0.1 to about 8 wt%, more preferably from about 0.2 to about 7 wt% (by total weight of fatty material based on the total weight of the dye composition)

When used, silicone is typically present as emulsified droplets having a mean droplet diameter (D3,2) of 4 micrometres or less. Preferably the mean droplet diameter (D3,2) is 1 micrometre or less, more preferably 0.5 micrometre or less, and most preferably 0.25 micrometre or less.

A suitable method for measuring the mean droplet diameter (D3,2) is by laser light scattering using an instrument such as a Malvern Mastersizer.

Preferably the silicone is non-volatile, meaning that it has a vapour pressure of less than 1000 Pa at 25°C.

Suitable silicones are polydiorganosiloxanes, in particular polydimethylsiloxanes (dimethicones), polydimethyl siloxanes having hydroxyl end groups (dimethiconols), amino-functional polydimethylsiloxanes (amodimethicones) and mixtures thereof. Suitable silicones for use in compositions of the invention are available as pre-formed silicone emulsions from suppliers such as Dow Corning and GE Silicones. The use of such pre-formed silicone emulsions is preferred for ease of processing and control of silicone particle size. Such pre-formed silicone emulsions will typically additionally comprise a suitable emulsifier, and may be prepared by a chemical emulsification process such as emulsion polymerisation, or by mechanical emulsification using a high shear mixer. Pre-formed silicone emulsions having a mean droplet diameter (D3,2) of less than 0.15 micrometers are generally termed microemulsions.

The amount of silicone may suitably range from 0.05 to 10 wt%, preferably from 0.2 to 8 wt% (by total weight silicone based on the total weight of the dye composition).

The amount of nitrobenzene direct dye will vary depending on the shade desired and the quantity and nature of the other components. Illustratively, the amount of nitrobenzene direct dye can vary from about 0.001 to about 2 wt% (by weight based on the total weight of the dye composition).

The pH of the dye composition is suitably in the range 4 to 8.

A particularly preferred dye composition for use in the invention comprises (by weight based on total weight):

from about 60 to about 95 wt% water,
from about 0.1 to about 10 wt% cationic surfactant,
from about 0.01 to about 10 wt% fatty material; and from about 0.001 to about 2 wt% nitrobenzene direct dye of general formula (I) as described above.

According to the method of the invention, the nitrobenzene direct dye (as described above) is topically applied

to the hair in the presence of dihydroxyacetone.

When applied to the hair in this manner, the weight ratio of nitrobenzene direct dye (as described above) to dihydroxyacetone generally ranges from about 1:2 to about 1:20, and preferably ranges from about 1:5 to about 1:15, e.g. about 1:10.

Dihydroxyacetone (1,3-dihydroxy-2-propanone) may be represented by the following general structural formula:

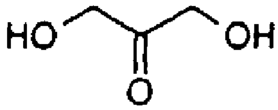

Preferably the dihydroxyacetone is provided in the form of a separate composition which is adapted for topical application to the hair together with the dye composition as described above.

Accordingly, the invention also provides a packaged "kit" for the non-oxidative (direct) coloration of hair, the kit comprising a dye composition as described above, in association with a second composition including dihydroxyacetone.

The amount of dihydroxyacetone included in the second composition generally ranges from about 1 to about 15 wt% and preferably ranges from about 2 to about 10 wt% (by weight based on the total weight of the second composition).

The second composition will preferably include further ingredients such as those specified above in relation to the dye composition, and in the amounts as specified above in relation to the dye composition.

A particularly preferred second composition for use in the invention comprises (by weight based on total weight):

from about 60 to about 95wt% water,
from about 0.1 to about 10 wt% cationic surfactant, from about 0.01 to about 10 wt% fatty material and from about 1 to about 15 wt% dihydroxyacetone.

Compositions for use in the invention can contain additional ingredients such as preservatives, chelating agents, fragrance and other substances customarily used in cosmetic hair colouring compositions.

Compositions for use in the invention do not require the incorporation of oxidising agents such as hydrogen peroxide to develop the colour. Accordingly it is preferred that such materials, if included at all, are present in minor quantities only, such as from 0 to 0.1 wt%, more preferably from 0 to 0.01 wt%, and most preferably from 0 to 0.001 wt% (by weight based on the total weight of the composition).

Compositions for use in the invention will desirably have a viscosity sufficient to prevent dripping when the composition is applied to the hair. Accordingly, the viscosity of compositions for use in the invention preferably ranges from 1,000 to 50,000 mPa.s (measured at 20° C. with a Brookfield rotation viscosimeter, with spindle 5 at 5 rpm).

The pH of compositions for use in the invention is suitably in the range 3.5 to 8.

In a typical method according to the invention, the compositions as described above are topically applied to wet or dry hair. Preferably the compositions are applied to freshly shampooed hair.

Compositions forming part of a "kit" as described above may be either mixed shortly before application to the hair, or applied successively to the hair without intermediate rinsing.

At the end of the colouring period, (generally about 5 to 45 minutes and preferably about 10 to 30 minutes), the hair is rinsed with plain water or a shampoo before being dried or left to dry. The dyeing temperature can vary from about room temperature, (e.g. about 20°) up to about 60°C and preferably ranges from about 20° to about 30°C.

The invention is further illustrated with reference to the following, non-limiting examples.

## EXAMPLES

A dihydroxyacetone containing hair conditioner formulation and a control (DHA free) hair conditioner formulation were prepared, having ingredients as shown in Table 1 below.

Table 1

| Ingredient | DHA Conditioner (% w/w) | Control Conditioner (% w/w) |
|---|---|---|
| Water | to 100 | to 100 |
| Lactic acid | 0.38 | 0.38 |
| Methyl parahydroxybenzoate | 0.2 | 0.2 |

(continued)

| Ingredient | DHA Conditioner (% w/w) | Control Conditioner (% w/w) |
|---|---|---|
| Stearyl alcohol | 5 | 5 |
| Behentrimonium chloride | 0.87 | 0.87 |
| Stearamidopropyl dimethylamine | 1.25 | 1.25 |
| Silicone emulsion | 2.5 | 2.5 |
| Dihydroxyacetone | 10 | -- |

A series of dye conditioner formulations were prepared, having ingredients as shown in Table 2 below.

Table 2

| Ingredient | Dye condr.1 (% w/w) | Dye condr.2 (% w/w) | Dye condr.3 (% w/w) | Dye condr.A (% w/w) | Dye condr.B (% w/w) | Dye condr.C (% w/w) |
|---|---|---|---|---|---|---|
| Water | to 100 | to 100 | to 100 | to 100 | to 100 | to 100 |
| Lactic acid | 0.34 | 0.34 | 0.34 | 0.34 | 0.34 | 0.34 |
| Methyl parahydroxybenzoate | 0.18 | 0.18 | 0.18 | 0.18 | 0.18 | 0.18 |
| Stearyl alcohol | 4.5 | 4.5 | 4.5 | 4.5 | 4.5 | 4.5 |
| Behentrimonium chloride | 0.78 | 0.78 | 0.78 | 0.78 | 0.78 | 0.78 |
| Stearamidopropyl dimethylamine | 1.125 | 1.125 | 1.125 | 1.125 | 1.125 | 1.125 |
| Silicone emulsion | 2.25 | 2.25 | 2.25 | 2.25 | 2.25 | 2.25 |
| 1,2-diamino-4-nitrobenzene | 2 | -- | -- | -- | -- | -- |
| 1,4-diamino-2-nitrobenzene | -- | 2 | -- | -- | -- | -- |
| 4-amino-3-nitrophenol | -- | -- | 2 | -- | -- | -- |
| Basic Red No.51 | -- | -- | -- | 2 | -- | -- |
| Acid Yellow No.3 | -- | -- | -- | -- | 2 | -- |
| Acid Blue No.9 | -- | -- | -- | -- | -- | 2 |

Dye conditioners 1 to 3 are examples of compositions for use in the method of the invention. Dye conditioners A to C are comparative examples.

Evaluation

Dye substantivity was evaluated by treating test hair switches with mixtures of the conditioner formulations described above. The components of each test treatment and their respective quantities are shown in Table 3 below.

Table 3

| Test treatmt. | Dye condr.1 | Dye condr.2 | Dye condr.3 | Dye condr. A | Dye condr. B | Dye condr. C | DHA condr. | Control condr. | Total |
|---|---|---|---|---|---|---|---|---|---|
| Dye condr.1 | 0.05g | -- | -- | -- | -- | -- | -- | 0.15g | **0.2g** |

(continued)

| Test treatmt. | Dye condr.1 | Dye condr.2 | Dye condr.3 | Dye condr. A | Dye condr. B | Dye condr. C | DHA condr. | Control condr. | Total |
|---|---|---|---|---|---|---|---|---|---|
| Dye condr.1 + DHA | 0.05g | -- | -- | -- | -- | -- | 0.1g | 0.05g | **0.2g** |
| Dye condr.2 | -- | 0.05g | -- | -- | -- | -- | -- | 0.15g | **0.2g** |
| Dye condr.2 + DHA | -- | 0.05g | -- | -- | -- | -- | 0.1g | 0.05g | **0.2g** |
| Dye condr.3 | -- | -- | 0.05g | -- | -- | -- | -- | 0.15g | **0.2g** |
| Dye condr.3 + DHA | -- | -- | 0.05g | -- | -- | -- | 0.1g | 0.05g | **0.2g** |
| Dye condr.A | -- | -- | -- | 0.05g | -- | -- | -- | 0.15g | **0.2g** |
| Dye condr.A + DHA | -- | -- | -- | 0.05g | -- | -- | 0.1g | 0.05g | **0.2g** |
| Dye condr.B | -- | -- | -- | -- | 0.05g | -- | -- | 0.15g | **0.2g** |
| Dye condr.B + DHA | -- | -- | -- | -- | 0.05g | -- | 0.1g | 0.05g | **0.2g** |
| Dye condr.C | -- | -- | -- | -- | -- | 0.05g | -- | 0.15g | **0.2g** |
| Dye condr.C + DHA | -- | -- | -- | -- | -- | 0.05g | 0.1g | 0.05g | **0.2g** |

Method

A wet bleached white human hair switch weighing 0.3g was treated with 0.2g of test treatment as defined in Table 3 above.

The treated switch was massaged for 1 minute, left for 10 minutes then rinsed for 60 seconds in tap water (flow rate 3Umin, ~20°C).

The hair switch was treated with 0.1 g shampoo formulation, massaged for 30 seconds and rinsed for 30 seconds. The described shampoo washing process was repeated.

0.2g of test treatment was applied again to the washed switch. The hair switch was massaged for 1 minute, left for 10 minutes then rinsed for 60 seconds in tap water (flow rate 3Umin, ~20°C). The hair switch was left to dry naturally and stored for 24 hours at 25°C. The colour of the switch was measured using a reflectometer and expressed as the CIE L*a*b* values.

The hair switch was wet with tap water then treated with 0.1 g shampoo formulation, massaged for 30 seconds and rinsed for 30 seconds. The described shampoo washing process was repeated and the switch dried naturally. The hair switch was wet with tap water again and treated with 0.1 g shampoo formulation, massaged for 30 seconds and rinsed for 30 seconds. The described shampoo washing process was repeated and the switch dried naturally. This process was repeated until the hair switch had been washed a total of 10 times. The colour of the switch was measured using a reflectometer and expressed as the CIE L*a*b* values.

The dyes coloured the hair and this was measured as the decrease in L* value relative to an undyed switch.

The substantivity of the dyes was measured as the colour loss on shampooing, which was calculated as follows:

Colour loss: • L = L (dyed pre-shampoo wash)- L(dyed post shampoo wash)

The results are shown below in Table 4. Lower values indicate increased substantivity and resistance to wash off. 95% confidence limits are also given.

| Test treatment | Colour loss • L ±95% confidence limit |
|---|---|
| Dye conditioner 1 | 4.07 ±0.62 |
| Dye conditioner 1 + DHA | 0.05 ±0.65 |
| Dye conditioner 2 | 8.25 ±0.82 |
| Dye conditioner 2 + DHA | 3.26 ±0.88 |
| Dye conditioner 3 | 8.83 ±0.89 |
| Dye conditioner 3 + DHA | 5.43 ±0.98 |
| Dye conditioner A | 3.63 ±0.93 |
| Dye conditioner A + DHA | 3.59 ±0.90 |
| Dye conditioner B | 5.62 ±0.63 |
| Dye conditioner B + DHA | 4.70 ±0.92 |
| Dye conditioner C | 0.71 ±0.85 |
| Dye conditioner C + DHA | 0.38 ±0.74 |

The above results demonstrate significantly reduced colour loss when DHA is combined with Dye conditioners 1 to 3 according to the method of the invention.

By contrast, no significant improvement in dye substantivity is found when DHA is combined with any of the comparative dye conditioners A,B and C.

## Claims

1. A method for the non-oxidative (direct) coloration of hair, the method comprising the step of topically applying a direct dye to the hair in the presence of dihydroxyacetone; the direct dye having a nitrobenzene chromophore and at least one primary amine ($-NH_2$) group.

2. A method according to claim 1, in which the primary amine group of the direct dye is directly bonded to the benzene nucleus.

3. A method according to claim 1 or 2, in which the direct dye is **characterised by** general formula (I):

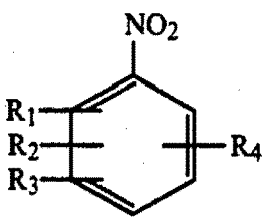

(I)

in which $R_1$ is $-NH_2$ or $-NHCH_2CH_2NH_2$ ;
in which $R_2$,$R_3$ and $R_4$ are each independently selected from hydrogen, halogen, -OH,$-NH_2$, $-(C_{1-4}$ alkyl), $-OR_5$,

$-NHR_5$ and $-N(R_5)_2$;
in which each $R_5$ is independently selected from $C_{1-4}$ alkyl, monohydroxy ($C_{1-4}$ alkyl), polyhydroxy ($C_{1-4}$ alkyl) and amino($C_{1-4}$ alkyl)groups;
and subject to the proviso that at least one of the substituents $R_2$,$R_3$ and $R_4$ is other than hydrogen.

**4.** A packaged kit for the non-oxidative (direct) coloration of hair, the kit comprising a dye composition comprising a direct dye having a nitrobenzene chromophore and at least one primary amine ($-NH_2$) group; in association with a second composition including dihydroxyacetone.

**5.** A kit according to claim 4, in which the amount of dihydroxyacetone included in the second composition ranges from 2 to 10 wt% (by weight based on the total weight of the second composition).

**6.** A kit according to claim 4 or 5, in which the dye composition comprises (by weight based on total weight):

from 60 to 95 wt% water,
from 0.1 to 10 wt% cationic surfactant,
from 0.01 to 10 wt% fatty material;
and from 0.001 to 2 wt% nitrobenzene direct dye of general formula (I).

**7.** A kit according to any one of claims 4 to 6, in which the nitrobenzene direct dye is selected from:

1,4-diamino-2-nitrobenzene,
1,2-diamino-4-nitrobenzene,
4-amino-3-nitrophenol,
2-amino-3-nitrophenol,
2-amino-6-chloro-4-nitrophenol,
1-amino-3-methyl-4-[(2-hydroxyethyl)amino]-6-nitrobenzene (HC Violet No. 1),
1-amino-4-[(2-hydroxyethyl)amino]-2-nitrobenzene (HC Red No. 7),
1-amino-4-[di-(2-hydroxyethyl)amino]-2-nitrobenzene hydrochloride (HC Red No. 13),
1-amino-5-chloro-4-[(2-hydroxyethyl)amino]-2-nitrobenzene,
4-amino-1-[(2-hydroxyethyl)amino]-2-nitrobenzene (HC Red No. 3),
1-[(2-aminoethyl)amino]-4-(2-hydroxyethoxy)-2-nitrobenzene (HC Orange No. 2),
1-amino-5-chloro-4-[(2,3-dihydroxypropyl)amino]-2-nitrobenzene (HC Red No. 10),
1-amino-2-[(2-hydroxyethyl)amino]-5-nitrobenzene (HC Yellow No. 5),
1-amino-2-methyl-6-nitrobenzene,
3-[(2-aminoethyl)amino]-1-methoxy-4-nitrobenzene hydrochloride (HC Yellow No. 9);
and mixtures thereof.

Europäisches Patentamt
European Patent Office
Office européen des brevets

# EUROPEAN SEARCH REPORT

Application Number
EP 14 19 9499

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2014/230162 A1 (WANG JINFANG [CN] ET AL) 21 August 2014 (2014-08-21)<br>* claims 1,7 *<br>----- | 1-7 | INV.<br>A61K8/35<br>A61K8/41<br>A61Q5/06 |
| Y | JP H01 149708 A (LION CORP)<br>12 June 1989 (1989-06-12)<br>* abstract *<br>----- | 1-7 | |
| Y,D | US 3 930 792 A (ALPERIN GEORGE ET AL)<br>6 January 1976 (1976-01-06)<br>* claims; examples *<br>----- | 1-7 | |
| A | DE 10 2011 017519 A1 (BEIERSDORF AG [DE])<br>31 October 2012 (2012-10-31)<br>* claims 1,10 *<br>----- | 1-7 | |

TECHNICAL FIELDS SEARCHED (IPC)

A61K
A61Q

The present search report has been drawn up for all claims

1

| Place of search | Date of completion of the search | Examiner |
|---|---|---|
| Munich | 22 May 2015 | Miller, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 19 9499

This annex lists the patent family members relating to the patent documents cited in the above-mentioned European search report. The members are as contained in the European Patent Office EDP file on
The European Patent Office is in no way liable for these particulars which are merely given for the purpose of information.

22-05-2015

| Patent document cited in search report | Publication date | Patent family member(s) | Publication date |
|---|---|---|---|
| US 2014230162 A1 | 21-08-2014 | AR 088001 A1<br>CN 103826702 A<br>EP 2760546 A2<br>US 2014230162 A1<br>WO 2013045382 A2 | 30-04-2014<br>28-05-2014<br>06-08-2014<br>21-08-2014<br>04-04-2013 |
| JP H01149708 A | 12-06-1989 | NONE | |
| US 3930792 A | 06-01-1976 | CA 1024071 A1<br>US 3930792 A | 10-01-1978<br>06-01-1976 |
| DE 102011017519 A1 | 31-10-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 3930792 A